# EUROPEAN PATENT SPECIFICATION

(11) **EP 0 839 037 B1**
(45) Date of publication and mention of the grant of the patent: **13.11.2002**
(21) Application number: 96923703.1
(22) Date of filing: 12.07.1996
(51) Int. Cl.: A61K 31/445, A61K 31/28, A61K 31/30, A61K 31/075, A61K 31/05, A61K 35/78, A61K 31/12

(54) **BIOPROTECTANT COMPOSITION, METHOD OF USE AND EXTRACTION PROCESS OF CURCUMINOIDS**
LEBENSCHÜTZENDE MITTEL, VERFAHREN ZU DEREN VERWENDUNG SOWIE EXTRAKTIONSVERFAHREN FÜR CURCUMINOIDE
COMPOSITION BIOPROTECTRICE, SON PROCEDE D'UTILISATION ET PROCEDE D'EXTRACTION DE CURCUMINOIDES

(30) Priority: 14.07.1995 US 1161 P
(43) Date of publication of application: 06.05.1998
(73) Proprietor: Sabinsa Corporation, Piscataway, NJ 08854 (US)
(72) Inventor: MAJEED, Muhammed, Piscataway, NJ 08854 (US); BADMAEV, Vladimir, Piscataway, NJ 08854 (US); RAJENDRAN, R., Bangalore 69 (IN)
(74) Representative: Huber, Bernhard, Dipl.-Chem.
(86) International application number: US9611431
(87) International publication number: WO97003674

(56) References cited:
- EP-A- 0 639 336
- WO-A-94/13743
- WO-A-96/03999
- DE-A- 2 924 345
- FR-A- 2 239 478
- FR-A- 2 343 433
- FR-A- 2 655 054
- GB-A- 2 184 341
- US-A- 4 263 333
- US-A- 4 719 111
- US-A- 5 108 750
- US-A- 5 120 538
- US-A- 5 266 344
- US-A- 5 401 504
- US-A- 5 401 777
- HUANG ET AL: "Effects of Curcumin, DM-Curcumin, BDM-Curcumin and Tetrahydrocurcumin on 12-O-Tetradecanoylphorbol-13-Acetate-Induc ed Tumor Promotion" CARCINOGENESIS, vol. 16, no. 10, 1995, pages 2493-2497, XP002087734
- TOENNESEN: "Studies on Curcumin and Curcuminoids" ZEITSCHRIFT FÜR LEBENSMITTELUNTERSUCHUNG UND -FORSCHUNG, vol. 194, no. 2, 1992, pages 129-130, XP002087735
- TODA ET AL: "Natural Antioxidants. III. Antioxidative Components Isolated from Rhizome of Curcuma Longa" CHEM. PHARMACEUT. BULL., vol. 33, 1985, pages 1725-1728, XP002087736
- GHOSH ET AL: "Antifungal Activity in Rhizomes of Curcuma Amada Roxb" INDIAN JOURNAL OF EXPERIMENTAL BIOLOGY, vol. 18, 1980, pages 174-176, XP002087737
- JOHRI ET AL: "An Ayurvedic Formulation "Trikatu" and its Constituents" JOURNAL OF ETHNOPHARMACOLOGY, vol. 37, 1992, pages 85-91, XP002087738
- KIUCHI: "Studies on Crude drugs Effective on Visceral Larva Migrans. XVI. Nematocidal Ectivity of Turmeric: Synergistic Action of Curcuminoids" CHEM. PHARM. BULLETIN, vol. 41, no. 9, 1993, pages 1640-1643, XP002087739
- BIOLOGICAL ABSTRACTS, Volume 100, No. 12, issued 1995, HUANG et al., "Effects of Curcumin, Demethoxycurcumin, Bisdemethoxycurcumin and Tetrahydrocurcumin on 12-O-Tetradecanoylphorbol-13-Acetate-Induce d Tumor Promotion", Abstract Number 187972; & CARCINOGENESIS, 16(10), 1995, pages 2493-2497.
- CHEM. PHARM. BULL., Volume 33, Number 4, issued 1985, TODA et al., "Natural Antioxidants. III. Antioxidative Components Isolated from Rhizome of Curcuma Longa L.", pages 1725-1728.
- JOURNAL OF ETHNO-PHARMACOLOGY, Volume 38, issued 1993, AMMON et al., "Mechanism of Antiinflammatory Actions of Curcumine and Boswellic Acids", pages 113-119.

## Description

### I. A novel concept of bioprotectant activity

Curcuma longa (Fam. Zingiberaccae) or Turmeric is one of the oldest herbs in Ayurveda materia medica, and has been used in Ayurveda medicine internally as a stomach, tonic and blood purifier, and topically in the prevention and treatment of skin diseases. The significance of turmeric in medicine has changed considerably since the very recent discovery of the anti-oxidant properties of naturally occurring phenolic compounds. The same ground dried rhizome of Curcuma longa, which has been used for centuries as a spice, food preservative and a coloring agent, has been found to be a rich source of phenolic compounds or curcuminoids. There are three main curcuminoids recognized, i.e., curcumin (diferuloylmethane), demethoxy curcumin (p-hydroxycinnamoyl[feruloyl]methane) and bis demethoxy curcumin (p,p-dihydroxydicinnamoylmethane).

Curcuminoids have scientifically documented anti-oxidant, anti-inflammatory, anti-bacterial, anti-fungal, antiparasitic, anti-mutagen, anti-cancer and detox properties. Their potential use in the prevention of cancer and in the treatment of infection with human immunodeficiency virus (HIV) are the subject of intensive laboratory and clinical research. Curcuminoids are recognized for their broad biological activity and safety of use. The biological activity of curcuminoids can best be described by the word "protective".

Curcumin and turmeric have been used as anti-inflammatory drugs for many years. More recently, there has been much interest in possible chemopreventive effects of curcumin. Curcumin inhibits tumor initiation by benxo[a]pyrene and tumor promotion by 12-0-tetradecanoylphorbol-13-acetate (TPA) in female CD-1 mice.

| THE BIOLOGICAL ACTIVITIES OF CURCUMIN | | |
|---|---|---|
| The biological activity | | Inhibition |
| 1. | TPA-induced epidermal ornithine decarboxylase activity | ++++ |
| 2. | TPA-induced edema of mouse ear | ++++ |
| 3. | TPA-induced epidermal hyperplasia | ++++ |
| 4. | TPA-induced epidermal DNA synthesis | + |
| 5. | Arachidonic acid-induced edema of mouse ear | +++ |
| 6. | Epidermal lipoxygenase activity | ++++ |
| 7. | Epidermal cyclooxygenase activity | ++++ |
| 8. | TPA-induced skin tumor promotion in previously initiated mice | ++++ |

Co-application of curcumin with TPA inhibits TPA-induced skin inflammation, increased epidermal ornithine decarboxylase activity. Huang et al. have also shown that 2% curcumin in the diet inhibits azoxymethane-induced foci in the colon of CF-1 mice and inhibits duodenal tumorigenesis in C57BL/6J mice previously initiated with N-ethyl-N'-nitro-N-nitrosguanidine.

Many of the anti-inflammatory and chemopreventive properties of curcumin may be related to the antioxidant properties of curcumin. In the present study, the antioxidant properties of curcumin, demethoxycurcumin, bisdemethoxycurcumin and several preparations containing varied proportions of curcumin, demethoxycurcumin, bisdemethoxycurcumin were measured by the Rancimat method.

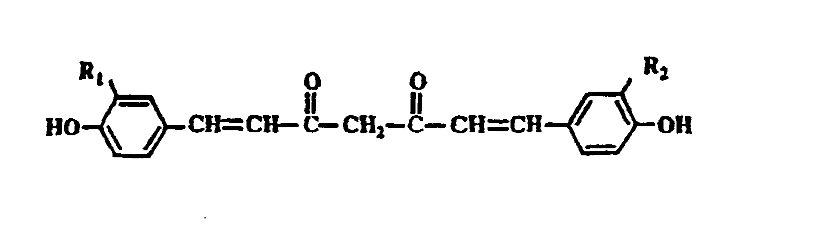

| R₁ | R₂ | |
|---|---|---|
| -OCH₃ | -OCH₃ | Curcumin |
| -H | -OCH₃ | Demethoxycurcumin |
| -H | -H | Bisdemethoxycurcumin |

The Rancimat method measures the conductivity changes caused by formation of small fatty acid molecules when the fats and oils are oxidized under elevated temperature and accelerated aeration. The induction times of lard with curcuminoids added were measured. The longer induction times suggest stronger antioxidant activity. All curcuminoids showed excellent antioxidant activity.

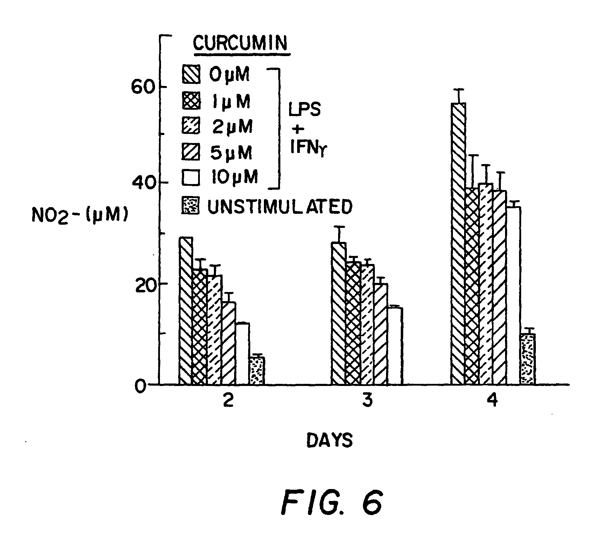

In chronic inflammation, cytokines induce the production of nitric oxide that is converted to DNA damaging and carcinogenic peroxynitrite and nitrite. The effect of curcumin on the generation of peroxynitrite radicals and nitrite was studied. Curcumin inhibited lipopolysaccharide (LPS) and interferon γ (INFγ) induced nitrite production by mouse peritoneal cells by more than 50% at 2.5 - 10uM (figure 6).

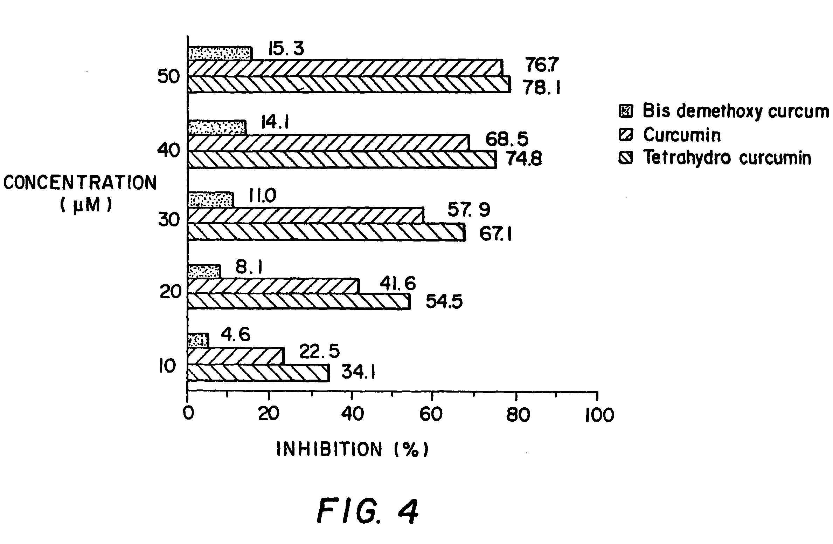

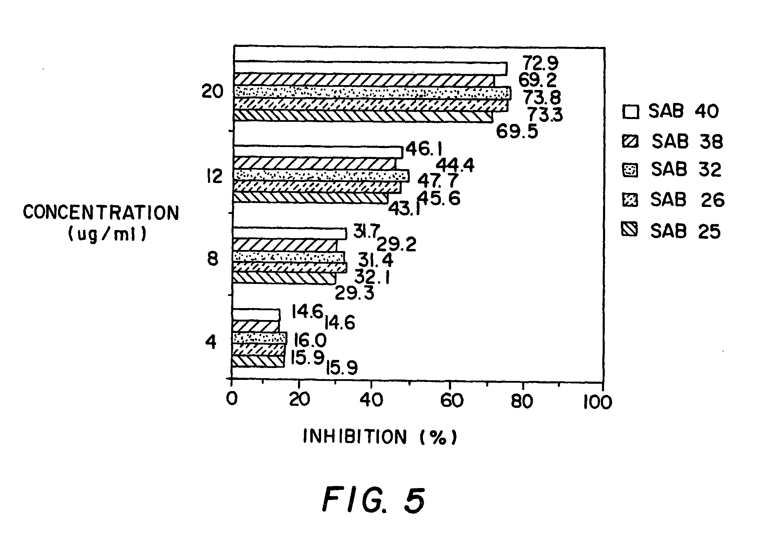

The present invention is directed to a composition containing three curcuminoids, i.e., curcumin, demethoxy curcumin and bis demethoxy curcumin, extracted from roots of turmeric. Curcuminoids possess distinct mechanisms which may explain their "protective" qualities. This protective activity has been defined by testing one of the best known properties of curcuminoids, their anti-oxidant properties. Two distinct modes of anti-oxidant action have been identified in curcuminoids: 1) prevention mode (Table 1) i.e. prevention of the formation of free radicals, and 2) intervention mode (Table 2) i.e. neutralizing action upon the already formed free radicals by the process of free-radical scavenging (figures 4 and 5).

**TABLE 1:**

| Prevention mode - the bioprotectant preventive activity of various compounds, as measured by the rancimat method [The rancimat method measures the time needed for inducing oxidative changes in the substrate - the longer the "induction time" the stronger the antioxidant properties]. Tested compounds were used in a standard concentration of 0.02% | |
|---|---|
| **SAMPLE** | **INDUCTION TIME IN HOURS** |
| CONTROL | 2.06 |
| BHT (common synthetic phenolic food additive) | 5.05 |
| CURCUMINOID COMPLEX Lot #4226 | 6.05 |
| GRAPE SEED EXTRACT Lot #3034 | 2.13 |
| PINE BARK EXTRACT Lot #1372 | 2.13 |

**Table 2:**

| Intervention mode - the bioprotectant intervention activity of curcuminoids, measured as DPPH free-radical scavenging ability against a control with "0" ability to scavenge free radicals. | | |
|---|---|---|
| **SAMPLE** | **CONCENTRATION ug/ml** | **SCAVENGING ABILITY %** |
| CURCUMINOID COMPLEX LOT NO. RD/CUR/02 | 4 | 16 |
| CURCUMINOID COMPLEX LOT NO. RD/CUR/02 | 8 | 31.4 |
| CURCUMINOID COMPLEX LOT NO. RD/CUR/02 | 12 | 47.7 |
| CURCUMINOID COMPLEX LOT NO. RD/CUR/02 | 20 | 73.8 |

The composition of the present invention showed significant activity in both anti-oxidant mechanisms studied (prevention and intervention). It should be noted that some other natural anti-oxidants, which have been screened together with curcuminoids, do not offer combined prevention and intervention activity. It has been concluded that the broad biological mechanism of curcuminoids is owed, to a large extent, to the combined mechanisms of prevention and intervention. Prevention and intervention result in the totality of protective qualities. Since curcuminoids may protect the integrity of biological systems by preventing the free-radical assault and by intervening to stop the assault, it is proposed to classify curcuminoids and any similar compound as a "bioprotectant".

The anti-oxidant potential of the composition was evaluated against the individual curcuminoids, i.e., curcumin, demethoxy curcumin, bis demethoxy curcumin, and other natural anti-oxidants like grape seed extract and pine bark extract *in vitro*. Interestingly, the present composition showed better bioprotectant activities than did the pure compounds as well as the two other natural anti-oxidants.

| THE OXIDATIVE INDUCTION TIME AND THE ANTIOXIDANT INDEX OF LARD WITH AND WITHOUT ANTIOXIDANT ADDED | | |
|---|---|---|
| Sample | Induction time(Hrs) | Antioxidant index |
| Control | 2.06 | |
| BHT (Synthetic phenolic) | 5.05 | 2.45 |
| Curcumin 99% Lot #001 | 2.83 | 1.37 |
| BDM Curcumin Lot #06 | 3.48 | 1.69 |
| Curcuminoids Lot #4226 | 6.05 | 2.92 |
| Grape Seed Ex. Lot #3034 | 2.13 | 1.03 |
| Pine Bark Ex. Lot #1372 | 2.13 | 1.03 |

| EFFECT OF CURCUMINOID COMPOSITION ON THE ANTIOXIDANT INDEX IN RANCIMAT METHOD | | | | |
|---|---|---|---|---|
| Sample | Curcumin % | BDM Curcumin % | DM Curcumin % | Antioxidant Index |
| SAB 25 | 78.6 | 2.2 | 16.7 | 2.7 |
| SAB 26* | 86.6 | 1.9 | 8.3 | 2.4 |
| SAB 32 | 80.2 | 2.5 | 15.5 | 2.0 |
| SAB 38* | 70.8 | 4.5 | 18.5 | 2.1 |
| SAB 40* | 67.2 | 2.8 | 14.8 | 1.5 |

| | | | | |
|---|---|---|---|---|
| * Samples SAB26, SAB38 and SAB40 do not form part of the invention | | | | |

### II. The unique formula for a curcuminoid composition

It has been found that the composition of curcuminoids may affect their bioprotectant activity, since individual components display different bioprotectant potential. For example, curcumin alone does not provide a strong prevention against free-radicals. On the other hand, curcumin shows strong intervention properties in neutralizing the already formed free-radicals. Similarly, a derivative of curcumin, tetrahydrocurcumin (THC), provides excellent intervention in scavenging already formed free-radicals, but is considerably less potent in the prevention of free-radical formation.

As a result of experimentally confirmed differences among various curcuminoid combinations, it has been concluded that only a carefully balanced composition of curcuminoids and derivatives thereof can result in optimal bioprotectant activity.

**Table 3:**

| Relationship between the composition of curcuminoid mixtures and the anti-oxidant activity as measured with the Rancimat method [The longer the induction time the better the anti-oxidant activity]. | | | | | |
|---|---|---|---|---|---|
| **Tested Compound** | **Curcumin %** | **DM Curcumin %** | **BDM Curcumin %** | **Total Curcumins** | **Induction Time (brs.)** |
| 1.Control | none | none | none | none | 2.00 |
| 2.Complex | 78.6 | 16.7 | 2.2 | 97.5 | 5.55 |
| 3. Complex* | 72.0 | 19.4 | 6.7 | 98.4 | 5.32 |
| 4. Complex* | 73.9 | 18.1 | 3.4 | 95.4 | 5.28 |
| 5.Complex | 75.1 | 17.8 | 2.3 | 95.2 | 5.20 |
| 6.Complex* | 74.2 | 20.6 | 3.1 | 97.9 | 5.08 |
| 7.Complex* | 72.9 | 18.9 | 4.7 | 96.5 | 5.07 |
| 8.Complex* | 86.6 | 8.3 | 1.9 | 96.8 | 4.80 |

| | | | | | |
|---|---|---|---|---|---|
| *Complexes 3,4,6,7 and 8 do not form part of the invention | | | | | |

The following observations were made pertaining to the relationship between the different compositions of curcuminoid combinations and their anti-oxidant activity:
* the mixture of curcuminoids is generally a more effective bioprotectant than either of the three components alone;
* the proportion of the individual components occurring in a mixture of curcuminoids is an important factor in determining the bioprotectant properties;
* the total content of curcuminoids is of secondary importance to the bioprotectant properties, primary importance being the presence of the three curcuminoids in the appropriate weight ratio of the individual components;
* the addition of certain derivatives of curcuminoids, e.g., tetrahydrocurcumin; cyclo curcumin; tumerin; curcumin complexed with metals like potassium, zinc, calcium, copper, chromium, vanadium; etc. may increase the bioprotectant activity of curcuminoids.

The following composition of curcuminoids is preferred to afford maximum bioprotectant activity:
1. Curcumin should be present in an amount of no less than 75% and no more than 81% of the total curcuminoids.
2. Demethoxy curcumin should be present in an amount of no less than 15% and no more than 19% of the total curcuminoids.
3. Bis demethoxy curcumin should be present in the amount of no less than 2.2% and no more than 6.5% of the total curcuminoids.
4. Additional ingredients may be present, e.g., tetrahydrocurcumin in an amount ranging from 1% to 5%; curcuminoids complexed with metals like potassium, zinc, copper, chromium, vanadium, calcium, etc. in an amount ranging from 1% to 5%; alkaloid piperine preferably in the form of Bioperine™ (subject of U.S. Application Serial No. 08/393,738 filed on February 24, 1995) in an amount ranging from 0.001% to 1%; cyclo curcumin in an amount ranging from 1% to 5%; and tumerin in an amount ranging from 0.1% to 0.5%.

The following are examples of preferred combinations of curcuminoids and other ingredients:
1. Curcumin 78.6%, demethoxy curcumin 16.7%, bis demethoxy curcumin 2.5%, tetrahydrocurcumin 1%, potassium curcumin 0.5%, Bioperine™ (piperine) 0.5%.
2. Curcumin 80.2%, demethoxy curcumin 15.5%, bis demethoxy curcumin 2.5%, tetrahydrocurcumin 1.8%.
3. Curcumin 75%, demethoxy curcumin 15%, bis demethoxy curcumin 6.5%, tetrahydrocurcumin 2%, Bioperine™ (piperine) 1%.
4. Curcumin 76.8%, demethoxy curcumin 16.1%, bis methoxy curcumin 6.1%.
5. Curcumin 78.6%, demethoxy curcumin 16.7%, bis demethoxy curcumin 2.5%, cyclo curcumin 1%, potassium curcumin 0.5%, Bioperine™ (piperine) 0.5%.
6. Curcumin 78.6%, demethoxy curcumin 16.7%, bis demethoxy curcumin 2.5%, tumerin 0.1%, potassium curcumin 0.5%, Bioperine™ (piperine) 0.5%.
7. Curcumin 80.2%, demethoxy curcumin 15.5%, bis demethoxy curcumin 2.5%, cyclo curcumin 1.8%.
8. Curcumin 75%, demethoxy curcumin 15%, bis demethoxy curcumin 6.5%, cyclo curcumin 2%, Bioperine™ (piperine) 1%.

The present invention is the optimal composition showing both prevention and intervention activity according to the above provided definition of bioprotectants.

### III. The unique manufacturing procedure

The specific combination of the ingredients in the preparation can be accomplished by way of a novel extraction procedure of curcuminoids from rhizomae of Curcuma longa. The curcuminoid combination can be isolated by:
a) drying and powdering tumeric rhizomae to produce a resulting powder,
b) extracting the resulting powder with a solvent at a temperature between 30-60°C to produce an extract,
c) concentrating said extract,
d) lowering the temperature of said concentrated extract to between 0-15°C to crystallize any curcuminoids present,
e) isolating any resulting crystals,
f) dissolving any isolated crystals of curcuminoids in a solvent at a temperature between 30-50°C,
g) lowering the temperature of the dissolved curcuminoids to a temperature between 0-15°C, and
h) isolating any curcuminoids present.

Examples of suitable solvents used to extract the powder are ethylene dichloride, methylene dichloride and ethyl acetate. The volume of solvent can be 3 to 9 volumes calculated on the dry weight of powdered turmeric rhizomae. The extraction step(b) can be repeated several times and the individual extracts combined before concentration. The extracts can be filtered and concentrated by distillation under vacuum at temperatures just under 50°C. Suitable solvents for dissolving the crystals of curcuminoids are C₁-C₆ alkyl ketone solvents preferably acetone, methyl ketone, etc. Steps d)-g) are critical for obtaining the desired composition of curcuminoids. As a result of this extraction procedure the unique composition of curcuminoids is obtained.

Characteristics of the composition of the invention
- Description:: orange yellow crystalline powder.
- Solubility:: slightly soluble in alcohol, soluble in acetone and in glacial acetic acid.
- Identification:: by UV absorption;
by boric acid test - dilute ethanolic solution after acidifying with hydrochloric acid gives a reddish color with boric acid.
- Melting range:: melts between 180 and 185°C.
- Loss on drying:: not more than 0.5% w/w.
- Assay by HPLC:: determines composition of curcuminoids within specified range.

### B. A method for complexation of curcuminoids with

metals is also disclosed Curcuminoids form complexes with nontoxic metals like calcium, zinc, magnesium, chromium, etc. Curcuminoids can be complexed with metals using the following steps:
a) dissolving at least one curcuminoid in a mixture of methanol and acetone,
b) heating the resulting mixture to 40 - 50°C,
c) preparing a metal solution by dissolving a metal in a solvent,
d) adding the metal solution to the solution of curcuminoid to produce a mixture,
e) adjusting the pH of the mixture to 7.5 - 9.5 to precipitate any metal complexes,
f) filtering any metal complexes which precipitate, and
g) drying the metal complexes.

An example of the above described process is given hereby for a calcium complex with curcuminoids. As discussed above, the above method is suitable to prepare other metal complexes with curcuminoids.
1. The 36.8 gm of curcuminoids is dissolved in a mixture of methanol and acetone.
2. The mixture is heated to 40 - 50°C for 1 - 2 hours.
3. Simultaneously, a solution of calcium chloride is prepared by dissolving 5.5 gm of calcium chloride in methanol. The solution is filtered.
4. Calcium chloride solution is added to the solution of curcuminoids and stirred for 2 - 3 hours.
5. The pH of the mixture is adjusted to 7.5 - 9.5 using ammonia to precipitate the metal complexes.
6. The precipitate is filtered and washed with water and finally rinsed with methanol.
7. The wet cake is dried at 70 - 80°C.
8. The yield is approximately 25 gm of calcium complex with curcuminoids.

The composition of the present invention can be administered alone, or it can be mixed with a pharmaceutically-acceptable carrier or diluent depending on the mode of administration. Oral administration is preferred, but parenteral and topical administration can be used. For oral administration, the composition of this invention can be used in the form of tablets, capsules, granules, powders, lozenges, syrups, elixirs, solutions, suspensions and the like, in accordance with the standard pharmaceutical practice.

For parenteral administration, which includes intramuscular, intraperitoneal, subcutaneous and intravenous use, sterile solutions of the active ingredients are usually prepared, and the pH of the solutions are suitably adjusted and buffered. For intravenous use, the total concentration of solutes should be controlled to render the preparation isotonic.

Carriers useful in formulating the preparations are commonly used pharmaceutically acceptable non-toxic carriers such as gelatin, lactose, sodium citrate, salts of phosphoric acid, starch, magnesium stearate, sodium lauryl sulphate, talc, polyethylene glycol etc. The carrier may be used with other additives such as diluents, binders, buffer agents, preservatives, sweetening agents, flavoring agents, glazes, disintegrators, coating agents, emulsifying agents, suspending agents, etc.

The daily dose of the preparation can be appropriately determined and is not particularly limited. However, in most instances, an effective dosage for an adult will be between 50-500mg/three times per os.

A further subject of the invention is the use of curcumin, demethoxy curcumin and bis demethoxy curcumin for the preparation of a medicament for preventing damage to a patient caused by free radicals, wherein said medicament neutralizes free radicals and prevents the formation of free radicals, comprising preparing a composition comprising the following components: curcumin, demethoxy curcumin, and bis demethoxy curcumin, wherein said components are purified individually or in combination and adjusted to the following ranges: 75-81 % curcumin, 15-19% demethoxy curcumin, and 2.2-6.5% bis demethoxy curcumin, based on the amount of the total curcuminoids. According to one specific embodiment said bis demethoxy curcumin is present in an amount of less than 5%.

### Brief Description of the Drawings

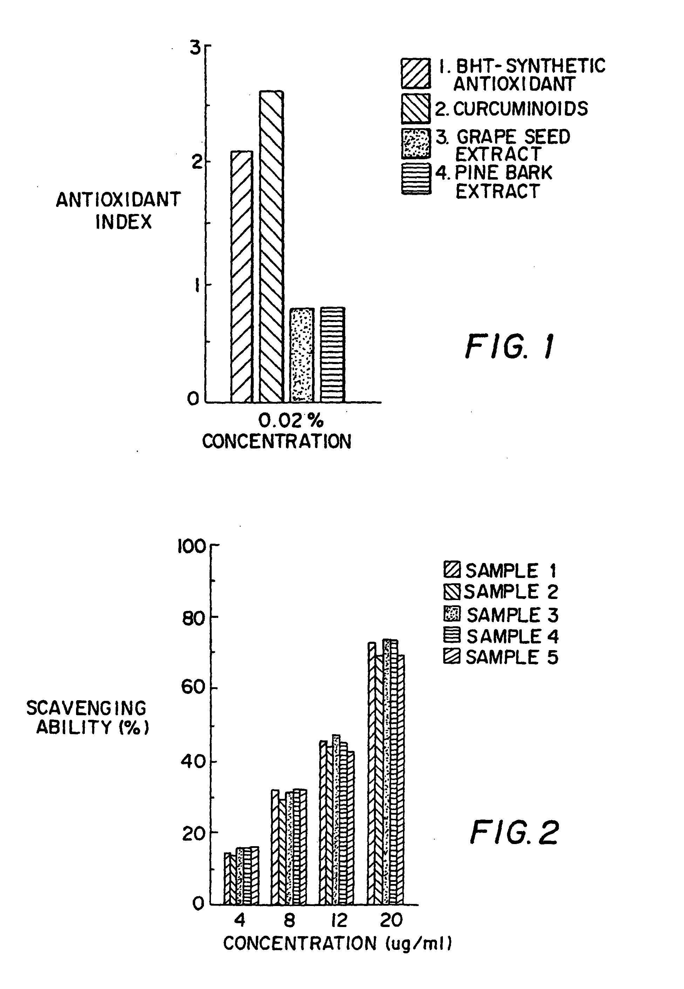

Figure 1 shows the results of an assay to determine the effectiveness of curcuminoids in the prevention of free radical formation (prevention mode). Free radical formation is measured using the Rancimat method.

Figure 2 shows the results of an assay to determine the effectiveness of curcuminoids in scavenging free radicals (intervention mode). The ability to scavenge free radicals is measured using the DPPH Radical Scavenging method.

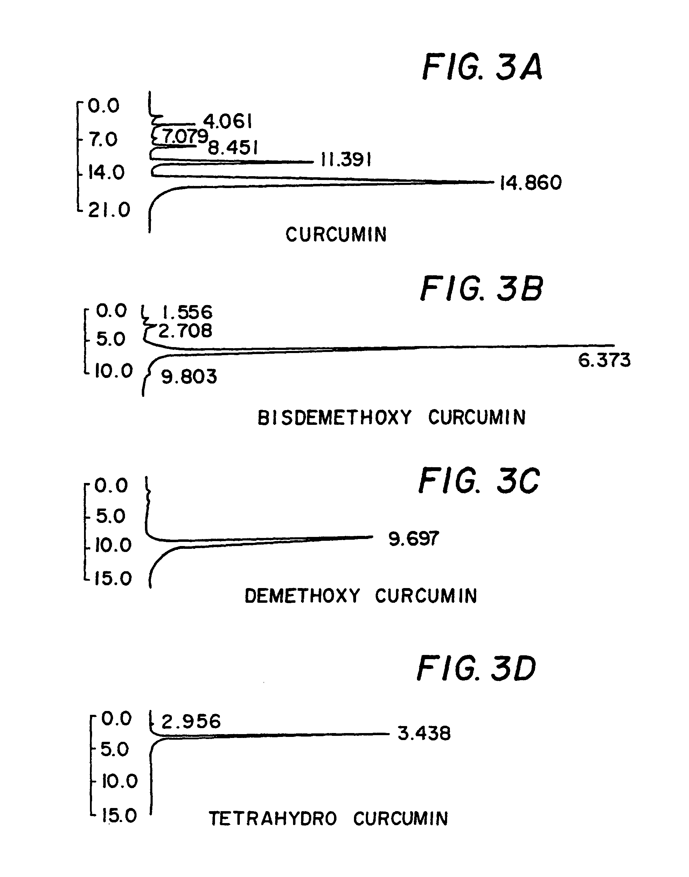

Figure 3 is an HPLC graph of the main components of the present composition.

Figure 4 shows the DPPH radical-scavenging ability of curcumin, tetrahydro curcumin, and bis demethoxy curcumin.

Figure 5 shows DPPH radical-scavenging ability of mixtures of curcuminoids.

Figure 6 shows the effect of curcumin on NO₂⁻ production.

### References

1. Ho, C.T., Chen, Q., Shi, H., Zhang, K.Q. and Rosen. R.T. (1992) "Antioxidative effect of polyphenol extract prepared from various Chinese tea", *Preventive Med.,* 21:520-525 [RANCIMAT METHOD].
2. Yen, G.C., Duh, P.D. (1994) "Scavenging effect of methanolic extracts of peanut hulls on free-radical and active-oxygen species", *J. Agric. Food Chem.,* 42:629-632 [DPPH RADICAL SCAVENGING].

## Claims

1. A bioprotectant composition, comprising the following components: curcumin, demethoxy curcumin, and bis demethoxy curcumin, wherein said components are purified individually or in combination and adjusted to the following ranges, based on the amount of the total curcuminoids: 75-81% curcumin, 15-19% demethoxy curcumin, and 2.2-6.5% bis demethoxy curcumin with the proviso that the ratio of curcumin : demethoxy curcumin : bis demethoxy curcumin is not 77.5 : 17.9 : 4.7 or 79.4 : 17.5 : 3.1.

2. The composition according to claim 1, further comprising at least one additional ingredient selected from the group consisting of tetrahydrocurcumin; curcuminoid complexed with a metal; alkaloid piperine; cyclo curcumin; and turmerin.

3. The composition according to claim 2, wherein said tetrahydrocurcumin; curcuminoid complexed with a metal; alkaloid piperine; cyclo curcumin; and turmerin if present are present in the following amounts, said tetrahydrocurcumin is present in an amount between 1-5%; said curcuminoid complexed with a metal is present in an amount between 1-5%; said alkaloid piperine is present in an amount between 0.001-1%; said cyclo curcumin is present in an amount between 1-5%; and said turmerin is present in an amount between 0.1-0.5%.

4. The composition according to claim 2, wherein said metal is selected from the group consisting of potassium, zinc, copper, chromium, vanadium and calcium.

5. The composition according to claims 1-4, further comprising a pharmaceutically acceptable carrier.

6. The use of curcumin, demethoxy curcumin and bis demethoxy curcumin for the preparation of a medicament for preventing damage to a patient caused by free radicals, wherein said medicament neutralizes free radicals and prevents the formation of free radicals in said patient, comprising preparing a composition comprising the following components: curcumin, demethoxy curcumin, and bis demethoxy curcumin, wherein said components are purified individually or in combination and adjusted to the following ranges: 75-81% curcumin, 15-19% demethoxy curcumin, and 2.2-6.5% bis demethoxy curcumin, based on the amount of the total curcuminoids.

7. The use according to claim 6, wherein said bis demethoxy curcumin is present in an amount less than 5%.

8. A method for preparing the curcuminoid composition according to claim 1, comprising the following steps:
a) drying and powdering turmeric rhizomae to produce a resulting powder,
b) extracting the resulting powder with a solvent at a temperature between 30-60°C to produce an extract,
c) concentrating said extract,
d) lowering the temperature of said concentrated extract to between 0-15°C to crystallize any curcuminoids present,
e) isolating any resulting crystals,
f) dissolving any isolated crystals of curcuminoids in a solvent at a temperature between 30-50°C,
g) lowering the temperature of the dissolved curcuminoids to a temperature between 0-15°C, and
h) isolating any curcuminoids present.

9. The method according to claim 8, wherein step b) is repeated at least once to produce additional extracts.

10. The method according to claim 8 or 9, wherein said extract is filtered before concentration.

11. The method according to claims 8-10, wherein said extract is concentrated by distillation under vacuum.

12. The method according to claims 8-11, wherein said crystals of curcuminoids are dissolved in a C₁-C₆ alkyl ketone solvent.

13. The method according to claim 12, wherein said C₁-C₆ alkyl ketone solvent is selected from the group consisting of acetone and methyl ketone.

14. The method according to claims 8-13, wherein said solvent is selected from the group consisting of ethylene dichloride, methylene dichloride and ethyl acetate.

## Patentansprüche

1. Bioprotektive Zusammensetzung, umfassend die folgenden Komponenten: Curcumin, Demethoxycurcumin und Bisdemethoxycurcumin, wobei die Komponenten jede für sich oder in Kombination aufgereinigt und auf die folgenden Bereiche eingestellt sind, bezogen auf die Menge der gesamten Curcuminoide: 75-81 % Curcumin, 15-19 % Demethoxycurcumin und 2,2-6,5 % Bisdemethoxycurcumin, mit der Maßgabe, dass das Verhältnis von Curcumin : Demethoxycurcumin : Bisdemethoxycurcumin nicht 77,5:17,9:4,7 oder 79,4:17,5:3,1 ist.

2. Zusammensetzung nach Anspruch 1, ferner umfassend mindestens einen weiteren Bestandteil ausgewählt aus der Gruppe bestehend aus Tetrahydrocurcumin; mit einem Metall komplexiertes Curcuminoid; Piperin-Alkaloid; Cyclocurcumin und Turmerin.

3. Zusammensetzung nach Anspruch 2, wobei das Tetrahydrocurcumin; das mit einem Metall komplexierte Curcuminoid; das Piperin-Alkaloid; das Cyclocurcumin und das Turmerin, wenn vorhanden, in den folgenden Mengen vorliegen: das Tetrahydrocurcumin liegt in einer Menge zwischen 1 bis 5% vor; das mit einem Metall komplexierte Curcuminoid liegt in einer Menge zwischen 1-5% vor; das Piperin-Alkaloid liegt in einer Menge zwischen 0,001-1% vor; das Cyclocurcumin liegt in einer Menge zwischen 1-5% vor, und das Turmerin liegt in einer Menge zwischen 0,1-0,5% vor.

4. Zusammensetzung nach Anspruch 2, wobei das Metall ausgewählt ist aus der Gruppe bestehend aus Kalium, Zink, Kupfer, Chrom, Vanadium und Calcium.

5. Zusammensetzung nach den Ansprüchen 1-4, ferner umfassend einen pharmazeutisch annehmbaren Träger.

6. Verwendung von Curcumin, Demethoxycurcumin und Bisdemethoxycurcumin, zur Herstellung eines Medikaments, um eine durch Radikale ausgelöste Schädigung eines Patienten zu verhindern, wobei das Medikament Radikale neutralisiert und die Bildung von Radikalen in dem Patienten verhindert, umfassend die Herstellung einer Zusammensetzung umfassend die folgenden Komponenten: Curcumin, Demethoxycurcumin und Bisdemethoxycurcumin, wobei die Komponenten jede für sich oder in Kombination aufgereinigt und auf die folgenden Bereiche eingestellt werden: 75-81% Curcumin, 15-19% Demethoxycurcumin und 2,2-6,5% Bisdemethoxycurcumin, bezogen auf die Menge der gesamten Curcuminoide.

7. Verwendung nach Anspruch 6, wobei das Bisdemethoxycurcumin in einer Menge von weniger als 5% vorhanden ist.

8. Verfahren zur Herstellung der Curcuminoid-Zusammensetzung nach Anspruch 1, umfassend die folgenden Schritte:
a) Trocknen und Pulverisieren von Curcuma-Rhizom, um daraus ein Pulver herzustellen,
b) Extrahieren des resultierenden Pulvers mit einem Lösungsmittel bei einer Temperatur zwischen 30-60 °C zur Herstellung eines Extrakts,
c) Aufkonzentrieren des Extrakts,
d) Erniedrigen der Temperatur des konzentrierten Extrakts auf zwischen 0-15 °C, um vorhandene Curcuminoide zu kristallisieren,
e) Isolieren der resultierenden Kristalle,
f) Lösen der isolierten Curcuminold-Krlstalle in einem Lösungsmittel bei einer Temperatur zwischen 30-50 °C,
g) Erniedrigen der Temperatur der gelösten Curcuminoide auf eine Temperatur zwischen 0-15 °C, und
h) Isolieren vorhandener Curcuminoide.

9. Verfahren nach Anspruch 8, wobei der Schritt b) mindestens einmal wiederholt wird, um weitere Extrakte herzustellen.

10. Verfahren nach Anspruch 8 oder 9, wobei der Extrakt vor dem Aufkonzentrieren filtriert wird.

11. Verfahren nach den Ansprüchen 8-10, wobei der Extrakt durch Destillation im Vakuum aufkonzentriert wird.

12. Verfahren nach den Ansprüchen 8-11, wobei die Curcuminoid-Kristalle in einem C₁-C₆-Alkylketön-Lösungsmittel gelöst werden.

13. Verfahren nach Anspruch 12, wobei das C₁-C₆-Alkylketon-Lösungsmittel ausgewählt wird aus der Gruppe bestehend aus Aceton und Methylketon.

14. Verfahren nach den Ansprüchen 8-13, wobei das Lösungsmittel ausgewählt wird aus der Gruppe bestehend aus Ethylendichlorid, Methylendichlorid und Ethylacetat.

## Revendications

1. Composition bio-protectrice, comprenant les composants suivants : curcumine, déméthoxycurcumine, et bis-déméthoxycurcumine, **caractérisée en ce que** lesdits composants sont purifés individuellement ou en combinaison et sont ajustés aux gammes suivantes, sur la base de la quantité des curcuminoïdes globaux : 75-81% de curcumine, 15-19% de déméthoxycurcumine, et 2,2-6,5% de bis-déméthoxycurcumine, sous réserve de ce que le rapport curcumine : déméthoxycurcumine : bis-déméthoxycurcumine n'est pas 77,5 : 17,9 : 4,7 ou 79,4 : 17,5 : 3,1.

2. Composition selon la revendication 1, comprenant en outre au moins un constituant supplémentaire sélectionné parmi le groupe se composant de la tétrahydrocurcumine ; d'un curcuminoïde complexé avec un métal ; de l'alcaloïde pipérine ; de la cyclocurcumine ; et de la turmérine.

3. Composition selon la revendication 2, **caractérisée en ce que** ladite tétrahydrocurcumine ; ledit curcuminoïde complexé avec un métal ; l'alcaloïde pipérine ; la cyclocurcumine ; et la turmérine, s'ils sont présents, sont présents dans les quantités suivantes, ladite tétrahydrocurcumine étant présente dans une quantité comprise entre 1-5 % ; ledit curcuminoïde complexé avec un métal étant présent dans une quantité comprise entre 1-5% ; ledit alcaloïde pipérine étant présent dans une quantité comprise entre 0,001-1% ; ladite cyclocurcumine étant présente dans une quantité comprise entre 1-5% ; et ladite turmérine étant présente dans une quantité comprise entre 0,1-0,5%.

4. Composition selon la revendication 2, **caractérisée en ce que** ledit métal est sélectionné parmi le groupe se composant du potassium, du zinc, du cuivre, du chrome, du vanadium et du calcium.

5. Composition selon les revendications 1-4, comprenant en outre un vecteur excipient acceptable du point de vue pharmaceutique.

6. Utilisation de la curcumine, de la déméthoxycurcumine et de la bis-déméthoxycurcumine en vue de la préparation d'un médicament pour empêcher des lésions chez un patient causées par des radicaux libres, **caractérisée en ce que** ledit médicament neutralise les radicaux libres et empêche la formation de radicaux libres chez ledit patient, comprenant la préparation d'une composition comprenant les composants suivants : curcumine, déméthoxycurcumine, et bis-déméthoxycurcumine, **caractérisée en ce que** lesdits composants sont purifés individuellement on en combinaison et sont ajustés aux gammes suivantes : 75-81% de curcumine, 15-19% de déméthoxycurcumine et 2,2-6,5% de bis-déméthoxycurcumine, sur la base de la quantité des curcuminoïdes globaux.

7. Utilisation selon la revendication 6, **caractérisée en ce que** ladite bis-déméthoxycurcumine est présente dans une quantité de moins de 5%.

8. Procédé de préparation de la composition de curcuminoïdes selon la revendication 1, comprenant les étapes suivantes :
a) le séchage et la pulvérisation de rhizomes de turméric pour produire la poudre résultante,
b) l'extraction de la poudre résultante à l'aide d'un solvant à une température comprise entre 30 et 60°C pour produire un extrait,
c) la concentration dudit extrait,
d) l'abaissement de la température dudit extrait concentré pour cristalliser, à une température comprise entre 0 et 15°C, un curcuminoïde quelconque présent,
e) l'isolement de tout cristal résultant,
f) la dissolution de tout cristal isolé de curcuminoïdes dans un solvant à une température comprise entre 30 et 50°C,
g) l'abaissement de la température des curcuminoïdes dissous à une température comprise entre 0 et 15°C, et
h) l'isolement de tout curcuminoïde présent.

9. Procédé selon la revendication 8, **caractérisé en ce que** l'étape b) est répétée à au moins une reprise pour produire des extraits supplémentaires.

10. Procédé selon la revendication 8 ou 9, **caractérisé en ce que** ledit extrait est filtré avant concentration.

11. Procédé selon les revendications 8-10, **caractérisé en ce que** ledit extrait est concentré par distillation sous vide.

12. Procédé selon les revendications 8-11, **caractérisé en ce que** lesdits cristaux de curcuminoïdes sont dissous dans un solvant d'alkylcétone en C₁-C₆.

13. Procédé selon la revendication 12, **caractérisé en ce que** ledit solvant d'alkylcétone en C₁-C₆ est sélectionné parmi le groupe se composant de l'acétone et de la méthylcétone.

14. Procédé selon les revendications 8-13, **caractérisé en ce que** ledit solvant est sélectionné parmi le groupe se composant du dichlorure d'éthylène, du dichlorure de méthylène et de l'acétate d'éthyle.
